# EUROPÄISCHE PATENTANMELDUNG

(11) **EP 4 703 423 A1**
(43) Veröffentlichungstag der Anmeldung: **04.03.2026**
(21) Anmeldenummer: 24198037.4
(22) Anmeldetag: 03.09.2024
(51) Int. Cl.: C08K 5/12, C08K 5/1515, C07C 13/18, C07C 61/08

(54) **WEICHMACHERZUSAMMENSETZUNG ENTHALTEND TRIMETHYLESTER DER 1,2,4-CYCLOHEXANTRIPROPIONSÄURE UND DEREN LÄNGERKETTIGE DERIVATE**

(71) Anmelder: Evonik Oxeno GmbH & Co. KG, 45772 Marl (DE)
(72) Erfinder: GRASS, Michael, 45721 Haltern am See (DE); RUSEK, Monika, 45149 Essen (DE); VAN EICKELS, Michael, 45657 Recklinghausen (DE)
(74) Vertreter: Evonik Patent Association

(57) **Zusammenfassung**

Die vorliegende Erfindung betrifft eine Weichmacherzusammensetzung enthaltend eine Verbindung der Formel (1) 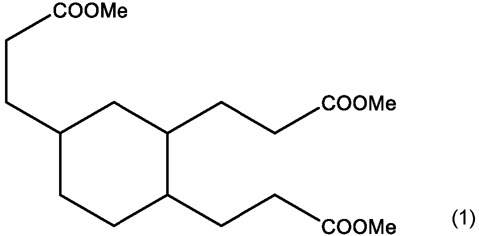 und mindestens einen längerkettigen Trialkylester der 1,2,4-Cyclohexantripropionsäure, wobei die drei Alkylgruppen im Trialkylester der 1 ,2,4-Cyclohexantripropionsäure jeweils 4 bis 9 Kohlenstoffatome aufweisen.

## Beschreibung

Gegenstand der Erfindung ist eine Weichmacherzusammensetzung enthaltend eine Verbindung der Formel (1) 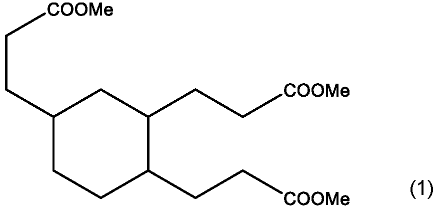 und mindestens einen längerkettigen Trialkylester der 1,2,4-Cyclohexantripropionsäure, wobei die drei Alkylgruppen im Trialkylester der 1,2,4-Cyclohexantripropionsäure jeweils 4 bis 9 Kohlenstoffatome aufweisen.

Die erfindungsgemäßen Verbindungen nach der Formel (1) sind Trimethylester der 1,2,4-Cyclohexantripropionsäure. Trimethylester der 1,2,4-Cyclohexantripropionsäure sind grundsätzlich bekannt und beispielsweise in der EP 3 838 886 A1 beschrieben worden. Dort wurde auch erwähnt, dass diese Ester der 1,2,4-Cyclohexantripropionsäure als Weichmacher eingesetzt werden können. Der Trimethylester wurde in diesem Zusammenhang als ein Produkt mit niedriger Geliertemperatur beschrieben.

Diese Ester zeigen beim Einsatz als Weichmacher in Kunststoffen nicht immer nur positive Eigenschaften. Würde man den Trimethylester nämlich als einzigen Weichmacher, beispielsweise in einer PVC-basierten Plastisol-Rezeptur einsetzen, würde das Plastisol eine recht hohe Viskosität aufweisen. Außerdem würde bei der Verarbeitung des Plastisols ein nicht unerheblicher Anteil des Trimethylesters verdampfen und somit nicht mehr im Endprodukt verbleiben. Es macht daher Sinn die erwähnten Trimethylester in Mischungen mit anderen Weichmachern einzusetzen.

Der vorliegenden Erfindung lag nun die Aufgabe zugrunde, eine Weichmacherzusammensetzung bereitzustellen, die Trialkylester der 1,2,4-Cyclohexantripropionsäure enthält, aber bessere anwendungstechnische Eigenschaften aufweist als Mischungen mit anderen Weichmachern.

Diese Aufgabe wird gelöst durch die Weichmacherzusammensetzung nach Anspruch 1. Gegenstand der Erfindung ist demnach eine Weichmacherzusammensetzung enthaltend eine Verbindung der Formel (1) 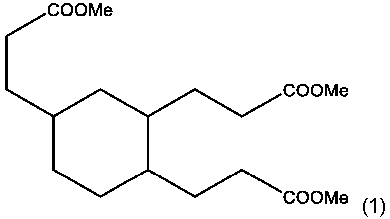 und mindestens einen längerkettigen Trialkylester der 1,2,4-Cyclohexantripropionsäure, wobei die drei Alkylgruppen jeweils 4 bis 9 Kohlenstoffatome aufweisen. Bevorzugte Ausführungsformen der vorliegenden Erfindung sind in den Unteransprüchen angegeben.

Der Trimethylester der 1,2,4-Cyclohexantripropionsäure, also die Substanz, die einen Teil der erfindungsgemäßen Weichmacherzusammensetzung darstellt, ist aktuell nicht kommerziell erhältlich. Ein Verfahren zur Herstellung dieser Ester wird aber beispielsweise in der EP 3 842 411 A1 beschrieben. Die Ester der 1,2,4-Cyclohexantripropionsäure sind somit allgemein zugänglich.

Neben den Trimethylestern der 1,2,4-Cyclohexantripropionsäure enthält die erfindungsgemäße Weichmacherzusammensetzung längerkettige Trialkylester der 1,2,4-Cyclohexantripropionsäure, wobei die drei Alkylgruppen jeweils unabhängig voneinander 4 bis 9 Kohlenstoffatome aufweisen. Die entsprechenden Verbindungen können durch die nachfolgende Markush-Formel beschrieben werden: 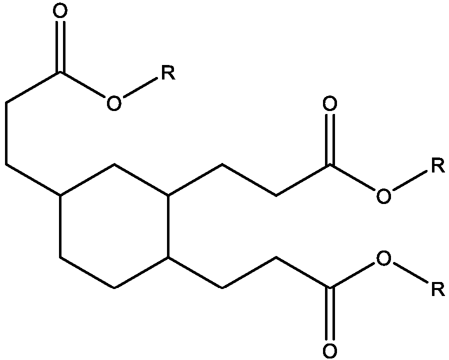 wobei die drei Reste R jeweils unabhängig voneinander Alkylgruppen mit 4 bis 9 Kohlenstoffatomen sind.

Die drei Alkylgruppen können dabei jeweils unabhängig voneinander aus der Gruppe, bestehend aus einer normalen Butylgruppe, einer Isobutylgruppe, einer normalen Pentylgruppe, einer 2-Methylbutylgruppe, einer 3-Methylbutylgruppe, einer normalen Hexylgruppe, einer Isohexylgruppe, einer normalen Heptylgruppe, einer Isoheptylgruppe, einer normalen Ocylgruppe, einer Isooctylgruppe, einer normalen Nonylgruppe oder einer Isononylgruppe, ausgewählt werden. Unter einer Isononylgruppe wird im Sinne der vorliegenden Erfindung eine Mischung aus linearen und verzweigten primären C9-Alkylgruppen verstanden.

In einer bevorzugten Ausführungsform der vorliegenden Erfindung weisen die drei Alkylgruppen der längerkettigen Trialkylester der 1,2,4-Cyclohexantripropionsäure jeweils die gleiche Anzahl an Kohlenstoffatomen auf. Das bedeutet, dass die drei Alkylgruppen 4, 5, 6, 7, 8 oder 9 Kohlenstoffatome aufweisen, aber nicht, dass die drei Alkylgruppen immer identisch sein müssen.

Es ist jedoch besonders bevorzugt, dass der längerkettige Trialkylester der 1,2,4-Cyclohexantripropionsäure in der erfindungsgemäßen Zusammensetzung ein Tripentylester der 1,2,4-Cyclohexantripropionsäure oder ein Triisononylester der 1,2,4-Cyclohexantripropionsäure ist. Unter den Begriff Tripentylester der 1,2,4-Cyclohexantripropionsäure fallen Trialkylester der 1,2,4-Cyclohexantripropionsäure, bei denen die jeder der drei Alkylgruppen eine 2-Methylbutylgruppe, eine 3-Methylbutylgruppe oder eine n-Pentylgruppe ist. Vorzugsweise beträgt der Anteil von n-Pentylgruppen mindestens 55 % und maximal 95% bezogen auf alle (= 100%) der Pentylgruppen im Tripentylester der 1,2,4-Cyclohexantripropionsäure. Unter den Begriff Triisononylester der 1,2,4-Cyclohexantripropionsäure fallen ebenso Mischester, die unterschiedliche Isononylgruppen enthalten. Grund dafür ist die Herstellung mittels kommerziell erhältlichem Isononanol (z. B. von der Evonik Oxeno Gmbh & Co. KG), das typischerweise eine Mischung unterschiedlicher C9-Alkohole (linear und verzweigt) darstellt.

Die Herstellung der erfindungsgemäßen längerkettigen Trialkylester der 1,2,4-Cyclohexantripropionsäure kann über die Umesterung des Trimethylesters der 1,2,4-Cyclohexantripropionsäure mit einem Alkohol mit 4 bis 9 Kohlenstoffatomen erfolgen oder über die direkte Veresterung der 1,2,4-Cyclohexantripropionsäure mit einem Alkohol mit 4 bis 9 Kohlenstoffatomen. Der Alkohol wird dabei im Überschuss eingesetzt, d. h. in einer Menge von mehr als 3 Mol pro Mol Esterkomponente, vorzugsweise 3,75 bis 5,25 mol Alkohol pro Mol Esterkomponente. Die Umesterung und die direkte Veresterung werden bevorzugt an einem sauren Katalysator durchgeführt, beispielsweise anorganische oder organische Brönsted- oder Lewis-Säuren.

Die beiden Substanzen können in unterschiedlichen Mengen in der erfindungsgemäßen Weichmacherzusammensetzung vorhanden sein. Es sollte klar sein, dass die beiden Substanzen in einer Menge vorhanden sein müssen, bei der sie eine Wirkung entfalten, d. h. wo eine weichmachende Wirkung eintritt. In einer bevorzugten Ausführungsform der vorliegenden Erfindung liegen die Verbindung nach Formel (1) und der längerkettige Trialkylester der 1,2,4-Cyclohexantripropionsäure in einem Gewichtsverhältnis (Verbindung nach Formel (1) : der längerkettige Trialkylester der 1,2,4-Cyclohexantripropionsäure) von 1 : 99 bis 99 : 1, vorzugsweise 10 : 90 bis 90 : 10, besonders bevorzugt 30 : 70 bis 70 : 30 in der Weichmacherzusammensetzung vor. In einer ganz besonders bevorzugten Ausführung der vorliegenden Erfindung liegen die Verbindung nach Formel (1) und der längerkettige Trialkylester der 1,2,4-Cyclohexantripropionsäure in einem Gewichtsverhältnis (Verbindung nach Formel (1) : der längerkettige Trialkylester der 1,2,4-Cyclohexantripropionsäure) von 30 : 70 bis 5 : 95 in der Weichmacherzusammensetzung vor.

Die erfindungsgemäße Weichmacherzusammensetzung der vorliegenden Erfindung kann zusätzlich ein epoxidiertes Öl oder einen epoxidierten Alkylester einer Fettsäure enthalten. Epoxidierte Öle oder entsprechende Ester können die thermische Stabilität und die mechanischen Eigenschaften verbessern.

Das epoxidierte Öl kann aus der Gruppe bestehend aus epoxidiertem Sojabohnenöl, epoxidiertem Rizinusöl, epoxidiertem Leinöl, epoxidiertem Palmöl, epoxidiertem Tallöl und Mischungen davon ausgewählt werden. Bevorzugt werden epoxidiertes Sojabohnenöl oder epoxidiertes Leinöl in der erfindungsgemäßen Weichmacherzusammensetzung eingesetzt. Besonders bevorzugt ist epoxidiertes Sojabohnenöl, das auch unter dem Akronym ESBO bekannt ist.

Die epoxidierten Fettsäureester können durch Umesterung der o.g. epoxidierten Öle mit Alkoholen im Bereich von 1 bis 10 Kohlenstoffatomen, bevorzugt 4 bis 9 Kohlenstoffatomen, hergestellt werden. Alternativ kann erst das natürliche Öl umgeestert und die Doppelbindungen der Fettsäure anschließend epoxidiert werden.

Das epoxidierte Öl oder die entsprechenden epoxidierten Fettsäurealkylester können in einer Menge von 1 bis 150 Gewichtsteilen bezogen auf 100 Gewichtsteile der Gesamtsumme aus der Verbindung gemäß Formel (1) und dem längerkettigen Trialkylester der 1,2,4-Cyclohexantripropionsäure eingesetzt werden. Das epoxidierte Öl oder die epoxidierten Fettsäureester können auch in Mengen von 2 bis 125 Gewichtsteilen, 5 bis 100 Gewichtsteilen, 10 bis 80 Gewichtsteilen oder 20 bis 70 Gewichtsteilen jeweils bezogen auf 100 Gewichtsteile der Gesamtsumme aus der Verbindung gemäß Formel (1) und dem längerkettigen Trialkylester der 1,2,4-Cyclohexantripropionsäure in der Weichmacherzusammensetzung enthalten sein.

Die erfindungsgemäße Weichmacherzusammensetzung kann zusätzlich noch mindestens einen weiteren Weichmacher enthalten, der aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, , Phthalaten, Trimellitaten, Cyclohexandicarboxylaten und oligomeren oder polymeren Estern auf Basis von Adipin-, Bernstein- oder Sebacinsäure, ausgewählt wird.

In einer bevorzugten Ausführungsform der vorliegenden Erfindung enthält die Weichmacherzusammensetzung mindestens einen weiteren Weichmacher, der aus der Gruppe, bestehend aus Alkylbenzoaten, Alkylsulfonsäureestern des Phenols, Dialkyladipaten, Glycerinestern, C4-bis C6-Alkansäureester von Polyolen, acetylierten oder nicht acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, Dialkylestern der 1,2-, 1,3- oder 1,4-Cyclohexandicarbonsäure.

In einer weiterhin bevorzugten Ausführungsform wird der mindestens ein weitere Weichmacher, der in der erfindungsgemäßen Weichmacherzusammensetzung enthalten sein kann, aus der Gruppe, bestehend aus C8- bis C13-Alkylbenzoaten, C4- bis C10-Dialkyladipaten, Pentaerythrit-tetravalerat, acetylierten oder nicht acetylierten Citronensäuretrialkylestern mit C2- bis C9-Alkylgruppen, C4- bis C10-Trialkyltrimellitaten, C4- bis C9-Dialkylterephthalaten, C4- bis C13-Dialkylphthalaten, insbesondere C9-bis C13-Dialkylphthalaten und C4- bis C10-Dialkylestern der 1,2-, 1,3- oder 1,4-Cyclohexandicarbonsäure, ausgewählt.

Besonders bevorzugt werden von den Citraten Butyl- oder Pentyl- oder höhere Citrate eingesetzt, die eine Acetylgruppe aufweisen können. Darunter fallen Tributylcitrat, Tripentylcitrat, Tri-n-hexylcitrat, Tri-2-ethylhexylcitrat, Triisononylcitrat, Acetyltributylcitrat und Acetyltripentylcitrat. Von den C4- bis C10-Dialkyladipaten sind Diethylhexyladipat (DEHA) und Diisononyladipat (DINA) bevorzugt. Von den C4- bis C9-Dialkylterephthalaten sind Dibutylterephthalat (DBT), Dipentylterephthalat (DPT) und Diethylhexylterephthalat (DEHT bzw. DOTP) bevorzugt. Von den C4- bis C10-Trialkyltrimellitaten sind Triethylhexyltrimellitat (TEHTM bzw. TOTM), und Triisononylterephthalat (TINTM) bevorzugt. Von den C4- bis C10-Dialkylestern der 1,4-Cyclohexandicarbonsäure sind der 1,4-Di-2-ethylhexylcyclohexandicarbonsäureester (1,4-DEHCH) und der 1,4-Diisononylcyclohexandicarbonsäureester (1,4-DINCH bzw. DINCD) bevorzugt. Von den C4- bis C10-Dialkylestern der 1,2-Cyclohexandicarbonsäure sind der 1,2-Di-2-ethylhexylcyclohexandicarbonsäureester (1,2-DEHCH) und der 1,2-Diisononylcyclohexandicarbonsäureester (1,2-DINCH) bevorzugt.

Ein weiterer Gegenstand der vorliegenden Erfindung ist eine Kunststoffzusammensetzung, die einen Kunststoff und die Weichmacherzusammensetzung, umfassend die Verbindung der Formel (1) und mindestens einen der längerkettigen Trialkylester der 1,2,4-Cyclohexantripropionsäure, wobei die drei Alkylgruppen jeweils 4 bis Kohlenstoffatome aufweisen, enthält. Die Kunststoffzusammensetzung kann zudem das epoxidierte Öl und /oder ein epoxidierten Fettsäurealkylester und/oder mindestens einen zusätzlichen Weichmacher aus der oben genannten Liste enthalten.

Geeignete Kunststoffe sind polymere Substanzen, die vorzugsweise aus der Gruppe, bestehend aus PVC (Polyvinylchlorid), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikonen, ausgewählt werden.

In einer bevorzugten Ausführungsform ist der Kunststoff in der Weichmacherzusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat. Besonders bevorzugt ist hiervon PVC als Kunststoff in der erfindungsgemäßen Kunststoffzusammensetzung.

Die Menge an der erfindungsgemäßen Weichmacherzusammensetzung in der Kunststoffzusammensetzung beträgt vorzugsweise 5 bis 150 Gewichtsteile, bevorzugt 10 bis 120 Gewichtsteile, besonders bevorzugt 15 bis 110 Gewichtsteile und ganz besonders bevorzugt 20 bis 100 Gewichtsteile pro 100 Gewichtsteile des Kunststoffs.

Die Kunststoffzusammensetzung kann neben den erwähnten Inhaltsstoffen zusätzliche Additive enthalten. Beispiele für zusätzliche Additive sind rheologische Additive, mit denen die Viskosität der Kunststoffzusammensetzung verringert werden kann. Beispiele für bekannte rheologische Additive sind die unter den Handelsnamen VISCOBYK^{®}-5120, VISCOBYK^{®}-5130 und VISCOBYK^{®}-4041 erhältlichen Produkte. Die Additive können in einem Anteil von 1 bis 12, bevorzugt 2 bis 10 Gewichtsteilen pro 100 Gewichtsteilen PVC in der Kunststoffzusammensetzung enthalten sein.

Darüber hinaus kann die Kunststoffzusammensetzung einen oder mehrere Thermostabilisator(en) enthalten. Geeignete Thermostabilisatoren sind Bleisalze, Organozinnverbindungen, Barium/Zinkverbindungen, Cadmiumverbindungen oder Zinkverbindungen, Calcium/Zink-Stabilisatoren und organisch-basierte Stabilisatoren (sog. OBS). Bevorzugte Thermostabilisatoren sind Barium/Zinkverbindungen, Calcium/Zink-Stabilisatoren und organisch-basierte Stabilisatoren (sog. OBS). Der Anteil des oder der Stabilisatoren in der Kunststoffzusammensetzung beträgt vorzugsweise 1 bis 6 Gewichtsteile pro 100 Gewichtsteilen PVC.

Als weitere Additive können darüber hinaus auch Füllstoffe, Pigmente, Treibmittel und Gleitmittel in der Kunststoffzusammensetzung enthalten sein.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen illustriert. Die folgenden Beispiele sollen die Erfindung erläutern, ohne deren Anwendungsbreite, die sich aus der Beschreibung und den Patentansprüchen ergibt, einzuschränken.

### Beispiele

### Beispiel 1a - Herstellung von Cyclohexan-1,2,4-tripropionsäuretrimethylester (Me-Tc)

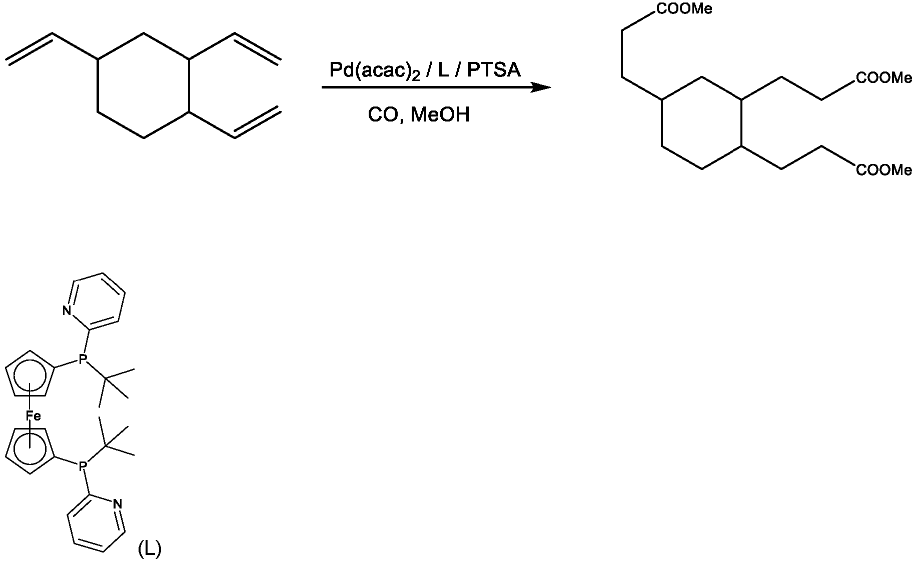

[Pd(acac)2] (15,2 mg, 0,1 mol%), der Katalysator L (103 mg, 0,4 mol%) und p-Toluolsulfonsäure (PTSA) (143 mg, 1,5 mol%) wurden unter einer Argonatmosphäre in einen 100-ml-Stahlautoklaven gegeben. Dann wurden MeOH (30 ml) und Trivinylcyclohexan (8,1 g, 50 mmol) mittels einer Spritze injiziert. Der Autoklav wurde dreimal mit CO gespült und anschließend mit einem CO-Druck von 40 bar beaufschlagt.

Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, 91% Ausbeute).

### Beispiel 1b - Herstellung von Cyclohexan-1,2,4-Tripropionsäure-tripentylester (MbPe-Tc)

Der nach Beispiel 1a hergestellte Trimethylester (Me-Tc) wurde in einer typischen Labor-Umesterungsapparatur mit 2-Methylbutanol und n-Pentanol (molares Verhältnis 7:93) zum Produkt MbPe-Tc umgeestert. Die Umesterungsapparatur besteht aus einem Glaskolben, der mit einem Rührer, Temperaturfühler, einer Destillationskolonne, Vakuumteiler und Destillationsaufbau ausgestattet ist.

Zunächst wurden 127 g 2-Methybutanol, 1690 g n-Pentanol und 1880 g M-Tc in der Umesterungsapparatur vorgelegt. Im Anschluss wurde die gesamte Apparatur mit Stickstoff gespült. und danach 2,72 g Tetra(n-butyl)titanat (TNBT) als Katalysator hinzugegeben. Danach wurde die Reaktion durch Aufheizen gestartet. Die Reaktionstemperatur betrug dabei 130 - 240°C, d. h. sie stieg während der Reaktion an. Methanol, dass bei der Umesterung entstand, wurde über die Kolonne und Destillationsapparatur aus dem System entfernt (65°C Kopftemperatur). Die C5-Alkohole, welche in der Kolonne vom Methanol getrennt wurden, wurden als Rückfluss wieder in das Reaktionsgemisch zurückgeführt. Als kein Destillatanfall mehr zu beobachten war, wurde der Reaktionsfortschritt über eine GC-Analyse kontrolliert Die Reaktion wurde beendet als der Triester >99 GC-Flächen% ausmachte, bezogen auf die Summe der Flächen des Edukts, Mono- Di- und Triesters

Im Anschluss an die Reaktion, wurde der überschüssige Alkohol bei 160 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Als nächstes wurde die Mischung bei 160 °C mit Stickstoff für 2 h gestrippt. Im letzten Schritt wird das Produkt über einen 0,5 µm PTFE-Filter und Perlite als Filterhilfsmittel klar filtriert.

### Beispiel 1c - Herstellung von Cyclohexan-1,2,4-tripropionsäure-trisononylester (In-Tc)

Der nach Beispiel 1a hergestellte Trimethylester (Me-Tc) wurde in einer typischen Labor Umesterungsapparatur analog wie in Beispiel 1b), nun aber mit Isononanol zum Produkt In-Tc umgeestert.

Zunächst wurden 1944 Isononanol (INA, Hersteller Evonik Oxeno GmbH & Co. KG) und 1230 g M-Tc in der Umesterungsapparatur vorgelegt. Im Anschluss wurde die gesamte Apparatur mit Stickstoff gespült. Als nächstes wurden 2,03 g Tetra(n-butyl)titanat (TNBT) als Katalysator hinzugegeben. Danach wurde die Reaktion durch Aufheizen gestartet. Die Reaktionstemperatur betrug dabei 130 - 240°C., d. h. sie stieg während der Reaktion an. Methanol, das bei der Umesterung entstand, , wurde über die Kolonne und Destillationsapparatur aus dem System entfernt (65°C Kopftemperatur). INA, dass in der Kolonne vom Methanol getrennt wurde, wurde als Rückfluss wieder in das Reaktionsgemisch zurückgeführt. Gelegentlich wurde leichtes Vakuum angelegt, um ausreichend Destillat zu erhalten. Als kein Destillatanfall mehr zu beobachten war, wurde der Reaktionsfortschritt über eine GC-Analyse kontrolliert.. Die Reaktion war beendet, als der Triester >99 GC-Flächen% ausmachte, bezogen auf die Summe der Flächen des Edukts, Mono- Di- und Triesters

Im Anschluss an die Reaktion, wurde der überschüssige Alkohol bei 180 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Als nächstes wurde die Mischung bei 180 °C mit Stickstoff für 2 h gestrippt. Im letzten Schritt wird das Produkt über einen 0,5 µm PTFE-Filter und Perlite als Filterhilfsmittel klar filtriert.

### Beispiel 2 - Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur sind jeweils in Gewichtsteilen (phr). Die Rezepturen der Polymerzusammensetzung ist in den Tabellen 1 und 2 aufgelistet.

**Tabelle 1: Übersicht über die hergestellten Plastisole (MbPe-Tc/Me-Tc).**

| Plastisol | 1* | 2 | 3 |
|---|---|---|---|
| | phr | phr | phr |
| PVC (Vestolit^{®} P 1430 K 70 Ultra; Fa. Vestolit) | 100 | 100 | 100 |
| Me-Tc (gem. Beispiel 1a) | 16,67 | | |
| MbPe-Tc (7:93) (gem. Beispiel 1b), (Mb: 2-Methylbutyl; Pe: *n*-Pentyl) | 33,33 | 33,33 | 33,33 |
| Eastman Effusion^{™} (Dibutylterephthalat, Fa. Eastman) | | 16,67 | |
| ELATUR^{®} DPT, (Di-pentylterephthalat, Fa. Evonik Oxeno & Co. KG) | | | 16,67 |
| epoxidiertes Sojabohnenöl als Co-Stabilisator (Edenol^{®} D81, Fa. Emery) | 3 | 3 | 3 |
| Thermostabilisator auf Ca/Zn-Basis (Reagens MBL 197/9 PF) | 2 | 2 | 2 |

| | | | |
|---|---|---|---|
| * = erfindungsgemäße Zusammensetzung phr = parts per hundred parts resin | | | |

**Tabelle 2: Übersicht über die hergestellten Plastisole (In-Tc/Me-Tc).**

| Plastisol | 4* | 5* | 6 | 7 | 8 | 9 |
|---|---|---|---|---|---|---|
| | phr | phr | phr | phr | phr | phr |
| PVC (Vestolit^{®} P 1430 K 70 Ultra; Fa. Vestolit) | 100 | 100 | 100 | 100 | 100 | 100 |
| Me-Tc /(gemäß Beispiel 1a) | 16,67 | 33,33 | | | | |
| In-Tc (In: Isononyl) (gem. Beispiel 1c) | 33,33 | 16,67 | 33,33 | 16,67 | 33,33 | 16,67 |
| Eastman Effusion^{™} (Dibutylterephthalat, Fa. Eastman) | | | 16,67 | 33,33 | | |
| ELATUR^{®} DPT, (Di-pentylterephthalat, Fa. Evonik Oxeno & Co. KG) | | | | | 16,67 | 33,33 |
| epoxidiertes Sojabohnenöl als Co-Stabilisator (Edenol^{®} D81, Fa. Emery) | 3 | 3 | 3 | 3 | 3 | 3 |
| Thermostabilisator auf Ca/Zn-Basis (Reagens MBL 197/9 PF) | 2 | 2 | 2 | 2 | 2 | 2 |

| | | | | | | |
|---|---|---|---|---|---|---|
| * = erfindungsgemäße Zusammensetzung | | | | | | |

Zuerst wurden die flüssigen und dann die pulverförmigen Bestandteile in einen PE-Becher eingewogen. Von Hand wurde die Mischung mit einem Spatel so eingerührt, dass kein unbenetztes Pulver mehr vorhanden war. Der verschlossene Becher wurde in die im Speedmixer befindliche Halterung gestellt und vermischt sowie entlüftet. Nach Beenden der Vermischung wurde die Temperatur des Plastisols mit Hilfe eines IR-Thermometers gemessen. Danach wurde das Plastisol sofort für weitere Untersuchungen in einem Klimaschrank bei 25,0 °C temperiert.

### Beispiel 3 - Gelierung der Plastisole

Die Untersuchung des Gelierverhaltens der Plastisole aus Beispiel 2 wurde mittels Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| | |
|---|---|
| Modus: | Temperatur-Gradient |
| Start-Temperatur: | 25 °C |
| End-Temperatur: | 180 °C |
| Heiz/Kühlrate: | 5 °C/min |
| Oszillations-Frequenz: | 4 bis 0,1 Hz Rampe logarithmisch |
| Kreisfrequenz Omega: | 10 s⁻¹ |
| Anzahl Messpunkte: | 63 |
| Messpunktdauer: | 0,5 min |
| Automatische Spaltnachführung F: | 0 N |
| Konstante Messpunktdauer | |
| Spaltweite | 0,5 mm |

### Durchführung der Messung

Auf die untere Messsystemplatte wurden mit dem Spatel einige Gramm der zu messenden Paste luftblasenfrei aufgetragen. Dabei wurde darauf geachtet, dass nach dem Zusammenfahren des Messsystems etwas Paste gleichmäßig aus dem Messsystem herausquellen konnte (nicht mehr als 6 mm rundum). Der Überschuss wurde mittels Spatel entfernt. Anschließend wurde die Temperierhaube über der Probe positioniert und die Messung gestartet. Bestimmt wurde die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Pastenviskosität von 1000 Pa*s verwendet. Die erhaltenen Ergebnisse sind in Tabelle 3 aufgeführt.

**Tabelle 3: Gelierung der Plastisole nach 24 Stunden, Temperatur in °C bei Erreichen einer Pastenviskosität von 1000 Pa*s.**

| Rezeptur | Weichmacherzusammensetzung nach Tabellen 1 und 2 | Geliertemperatur [°C] |
|---|---|---|
| 1* | MbPe-Tc : Me-Tc (2:1) | 69,3 |
| 2 | MbPe-Tc : DBT (2:1) | 69,8 |
| 3 | MbPe-Tc : DPT (2:1) | 72,7 |
| 4* | In-Tc : Me-Tc (2:1) | 78,2 |
| 5* | In-Tc : Me-Tc (1:2) | 67,2 |
| 6 | In-Tc : DBT (2:1) | 82,8 |
| 7 | In-Tc : DBT (1:2) | 68,0 |
| 8 | In-Tc : DPT (2:1) | 87,7 |
| 9 | In-Tc : DPT (1:2) | 74,8 |

| | | |
|---|---|---|
| * Versuch mit erfindungsgemäßer Weichmacherzusammensetzung | | |

Im Vergleich mit anderen Schnellgelierern sorgt der Einsatz des Cyclohexan-1,2,4-tripropionsäure-trimethylesters (Me-Tc) in den Weichmacherzusammensetzungen für eine deutliche Reduktion der Geliertemperatur. Wird mehr Me-Tc eingesetzt, kann die Geliertemperatur weiter gesenkt werden.

### Beispiel 4 - Herstellung von Folien

Die im Beispiel 2 hergestellten Plastisole wurden jeweils zu 1 mm dicken Folien verarbeitet. Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssiges Plastisol aufzufangen. Danach wurde das Plastisol vor der Rakel aufgetragen und diese durch Ziehen des Führungsrahmens mit der Rakel über das eingespannte Trennpapier verstrichen (3 m/min Geschwindigkeit). Danach wurde die Rakel herausgenommen und der Klebestreifen mit dem überschüssigen Plastisol abgenommen. Anschließend wurde der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 5 - Bestimmung des Masseverlusts (Aktivkohle-Verfahren)

In Anlehnung an DIN EN ISO 176 (Verfahren B) wurden aus den hergestellten PVC- Folien jeweils drei kreisrunde Scheiben mit einem Durchmesser von 5 cm ausgestanzt und zunächst für 16 Stunden im Exsikkator bei Raumtemperatur konditioniert. Auf der Analysenwaage wurden die Proben-Kreise ausgewogen, in einen Drahtkorb gelegt, welcher mit einer Klammer verschlossen wurde. Die Drahtkörbe wurden anschließend in eine mit Aktivkohle gefüllte Weißblechdose so platziert, dass sich zwischen den einzelnen Körben bzw. Korb und Deckel (gelocht) bzw. Boden jeweils etwa 130 ml Aktivkohle befanden.

Die befüllten Weißblechdosen wurden im vortemperierten Heizschrank (120 °C) so platziert, dass der Lüfter im Schrank nicht zugestellt wurde und die Dosen sich nicht berührten. Luftwechsel und Abluftklappe wurden auf jeweils 10 % eingestellt. Nach 72 Stunden wurden die Dosen wieder aus dem Schrank entnommen, abgekühlt, danach die einzelnen Proben den Körben entnommen, im Exsikkator nochmals 16 Stunden konditioniert und danach auf der Analysenwaage zurückgewogen. Die erhaltene Massedifferenz entsteht durch den Verlust an Weichmacher. Von den jeweils drei Massedifferenzen wurde der Mittelwert gebildet und prozentual der Verlust an Weichmacher errechnet. In Tabelle 4 sind die Ergebnisse der Flüchtigkeiten aufgeführt.

**Tabelle 4: Massenverlust der Prüfkörper nach drei Tagen bei 120 °C.**

| Foliennr. | Weichmacherzusamme nsetzung nach Tabellen 1 und 2 | Massenverlust nach 3 Tagen in % |
|---|---|---|
| 1* | MbPe-Tc : Me-Tc (2:1) | 3,5 |
| 2 | MbPe-Tc : DBT (2:1) | 11,0 |
| 3 | MbPe-Tc : DPT (2:1) | 7,8 |
| 4* | In-Tc : Me-Tc (2:1) | 3,4 |
| 5* | In-Tc : Me-Tc (1:2) | 5,4 |
| 6 | In-Tc : DBT (2:1) | 10,3 |
| 7 | In-Tc : DBT (1:2) | 20,3 |
| 8 | In-Tc : DPT (2:1) | 7,8 |
| 9 | In-Tc : DPT (1:2) | 14,4 |

| | | |
|---|---|---|
| * = erfindungsgemäße Zusammensetzung | | |

Der Massenverlust von Folien mit der erfindungsgemäßen Weichmacherzusammensetzung ist geringer als der von Folien, die mit Weichmacherzusammensetzungen, die andere Schnellgelierer enthalten, hergestellt worden sind.

## Patentansprüche

1. Weichmacherzusammensetzung enthaltend eine Verbindung der Formel (1) 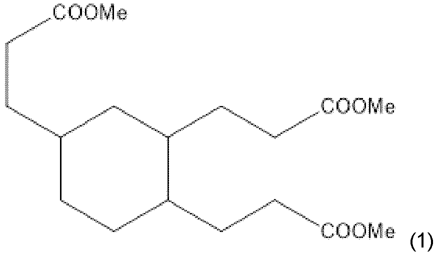 und mindestens einen längerkettigen Trialkylester der 1,2,4-Cyclohexantripropionsäure, wobei die drei Alkylgruppen jeweils 4 bis 9 Kohlenstoffatome aufweisen.

2. Weichmacherzusammensetzung nach Anspruch 1, wobei die drei Alkylgruppen jeweils unabhängig voneinander aus der Gruppe, bestehend aus einer normalen Butylgruppe, einer Isobutylgruppe, einer normalen Pentylgruppe, einer 2-Methylbutylgruppe, einer 3-Methylbutylgruppe, einer normalen Hexylgruppe, einer Isohexylgruppe, einer normalen Heptylgruppe, einer Isoheptylgruppe, einer normalen Ocylgruppe, einer Isooctylgruppe, einer normalen Nonylgruppe oder einer Isononylgruppe, ausgewählt werden.

3. Weichmacherzusammensetzung nach Anspruch 1 oder 2, wobei die drei Alkylgruppen des Trialkylesters der 1,2,4-Cyclohexantripropionsäure die gleiche Anzahl an Kohlenstoffatomen aufweisen.

4. Weichmacherzusammensetzung nach einem der vorhergehenden Ansprüche, wobei der längerkettige Trialkylester der 1,2,4-Cyclohexantripropionsäure ein Tripentylester der 1,2,4-Cyclohexantripropionsäure oder ein Triisononylester der 1,2,4-Cyclohexantripropionsäure ist.

5. Weichmacherzusammensetzung nach einem der vorhergehenden Ansprüche, wobei die Verbindung nach Formel (1) und der längerkettige Trialkylester der 1,2,4-Cyclohexantripropionsäure in einem Gewichtsverhältnis (Verbindung nach Formel (1) : 1,2-Dialkylcyclohexandicarbonsäureester) von 1 : 99 bis 99 : 1, vorzugsweise 10 : 90 bis 90 : 10, besonders bevorzugt 30 : 70 bis 70 : 30 in der Weichmacherzusammensetzung vorhanden sind.

6. Weichmacherzusammensetzung nach einem der vorhergehenden Ansprüche, wobei die Weichmacherzusammensetzung zusätzlich ein epoxidiertes Öl oder einen epoxidierten Alkylester einer Fettsäure enthält.

7. Weichmacherzusammensetzung nach Anspruch 6, wobei das epoxidierte Öl aus der Gruppe bestehend aus epoxidiertem Sojabohnenöl, epoxidiertem Rizinusöl, epoxidiertem Leinöl, epoxidiertem Palmöl, epoxidiertem Stearat, epoxidiertem Oleat, epoxidiertem Tallöl, epoxidiertem Linoleat und Mischungen davon ausgewählt wird.

8. Weichmacherzusammensetzung nach Anspruch 7, wobei das epoxidierte Öl epoxidiertes Sojabohnenöl oder epoxidiertes Leinöl, vorzugsweise epoxidiertes Sojabohnenöl ist.

9. Weichmacherzusammensetzung nach einem der Ansprüche 6 bis 8, wobei das epoxidierte Öl in einer Menge von 1 bis 150 Gewichtsteilen bezogen auf 100 Gewichtsteile der Gesamtsumme aus der Verbindung gemäß Formel (1) und des längerkettigen Trialkylester der 1,2,4-Cyclohexantripropionsäure in der Weichmacherzusammensetzung vorhanden ist.

10. Weichmacherzusammensetzung nach einem der vorherigen Ansprüche, wobei die Zusammensetzung zusätzlich noch einen weiteren Weichmacher aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, Trimellitaten, Cyclohexandicarboxylaten und oligomeren oder polymeren Estern auf Basis von Adipin-, Bernstein- oder Sebacinsäure, ausgewählt werden.

11. Kunststoffzusammensetzung, umfassend die Weichmacherzusammensetzung nach einem der Ansprüche 1 bis 10 und einen Kunststoff.

12. Kunststoffzusammensetzung nach Anspruch 11, wobei der Anteil der Weichmacherzusammensetzung 5 bis 150 Gewichtsteile pro 100 Gewichtsteile Kunststoff beträgt.

13. Kunststoffzusammensetzung nach Anspruch 11 oder 12, wobei der Kunststoff aus der Gruppe, bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat, ausgewählt wird.

14. Kunststoffzusammensetzung nach Anspruch 13, wobei der Kunststoff Polyvinylchlorid (PVC) ist.

15. Verwendung der Kunststoffzusammensetzung nach einem der Ansprüche 11 bis 14 in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.
